# EUROPÄISCHE PATENTSCHRIFT

(11) **EP 1 400 213 B1**
(45) Veröffentlichungstag und Bekanntmachung des Hinweises auf die Patenterteilung: **12.09.2007**
(21) Anmeldenummer: 03017086.4
(22) Anmeldetag: 28.07.2003
(51) Int. Cl.: A61B 17/58, A61F 2/46

(54) **Vorrichtung zum Applizieren von Knochenzement**
Apparatus for delivery of bone cement
Outil pour l'application de ciment osseux

(30) Priorität: 18.09.2002 DE 10243357
(43) Veröffentlichungstag der Anmeldung: 24.03.2004
(73) Patentinhaber: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE)
(74) Vertreter: Manitz, Finsterwald & Partner GbR

(56) Entgegenhaltungen:
- WO-A-01/93787
- WO-A-02/00143
- WO-A-03/000121
- WO-A-03/017854
- US-A- 4 269 331
- US-A- 4 405 249
- US-A- 4 671 263
- US-A- 4 832 692
- US-A- 5 370 221
- US-A1- 2003 014 056

## Beschreibung

Die vorliegende Erfindung betrifft eine Vorrichtung zum Applizieren von Knochenzement gemäß dem Oberbegriff des Anspruchs 1.

Applikationsvorrichtungen dieser Art werden verwendet, wenn Knochenstrukturen beispielsweise durch Knochenkrebs oder durch Osteoporose zersetzt oder brüchig geworden sind. Mit entsprechenden Vorrichtungen ist die Applikation von Knochenzement unmittelbar in die betroffenen Knochenstrukturen möglich, wodurch diese verfestigt werden.

Bei der Applikation sind dabei mehrere Anforderungen zu beachten. Zum einen muss die Befüllung der Applikationsvorrichtung sowie die Applikation in die betroffenen Knochenstrukturen sehr schnell, innerhalb weniger Minuten erfolgen, da die üblicherweise verwendeten Knochenzemente 6 bis 7 Minuten nach dem Anrühren auszuhärten beginnen. Zum anderen muss der Knochenzement mit sehr hohem Druck appliziert werden, da andernfalls eine ausreichende Durchdringung der Knochenstrukturen nicht gewährleistet ist. Letztlich muss die Applikation des Knochenzements gut steuerbar sein, da insbesondere bei der Applikation im Bereich der Wirbelsäule ein Fehlleiten des Knochenzements zu irreversiblen Schädigungen beispielsweise von Nerven führen kann.

Eine Vorrichtung zum Applizieren von Knochenzement ist aus der DE 100 64 202 A bekannt. Bei dieser Vorrichtung ist die Verschiebung des Kolbens zwischen einer Verschiebung durch eine Schraubbewegung und einer direkten Verschiebung in Längsrichtung ohne Schraubbewegung umstellbar. Durch diese Umstellbarkeit ist es möglich, dass beispielsweise das Auffüllen der Kammer durch eine Aufziehbewegung des Kolbens, das heißt eine direkte Verschiebung des Kolbens in Längsrichtung in sehr kurzer Zeit erfolgt. In umgekehrter Weise kann anschließend der in der Kammer vorhandene, flüssige Knochenzement durch direktes Vorschieben des Kolbens in kurzer Zeit so lange appliziert werden, bis der entstehende Gegendruck so groß wird, dass er durch die direkte Vorschubbewegung nicht mehr überwunden werden kann. In diesem Moment wird die Applikationsvorrichtung auf einen Modus "Verschieben des Kolbens durch Schraubbewegung" umgestellt, da durch die Schraubbewegung ein wesentlich höherer Druck auf den Kolben und damit auf den zu applizierenden Knochenzement ausgeübt werden kann als mit einer direkten Vorschubbewegung.

Vorrichtungen dieser Art haben sich in der Praxis bewährt. Da nach der vollständigen Applikation des Knochenzements der in der Vorrichtung noch verbleibende restliche Knochenzement üblicherweise sehr schnell aushärtet, sind diese Vorrichtungen bisher als Einwegvorrichtungen ausgebildet, das heißt nach der Applizierung des Knochenzements wird die gesamte Vorrichtung entsorgt, da eine Reinigung der Vorrichtung gemäß den klinischen Vorschriften praktisch nicht bzw. nur mit nicht vertretbarem finanziellen Aufwand möglich ist.

Aufgrund des fortschreitenden Kostenbewusstseins im Gesundheitswesen sowie aufgrund ökologischer Gesichtspunkte ist es jedoch wünschenswert, Vorrichtungen der eingangs genannten Art zumindest teilweise auch wieder verwendbar auszubilden.

Aus der WO 01/93787 A (Basis für den Oberbegriff des Anspruchs 1.) ist eine Vorrichtung der eingangs genannten Art bekannt. Bei dieser Vorrichtung besitzt der Kolben einen Rastansatz, über den er mit dem distalen Ende des Schafts verrastbar ist. Eine ähnliche Vorrichtung, bei der der Kolben ebenfalls mit dem Schaft verrastbar ist, ist auch aus der WO 02/00143 A bekannt. Letztlich ist aus der US-A-4 832 692 eine Vorrichtung zur Balloninsufflation bekannt, mit der der Ballon eines Ballonkatheters mit Flüssigkeit gefüllt und auf seinen gewünschten Außendurchmesser gebracht werden kann. Bei dieser Vorrichtung ist ein Kolben verdrehbar, jedoch nicht abnehmbar an dem distalen Ende eines entsprechenden Schafts angeordnet.

Es ist daher eine Aufgabe der vorliegenden Erfindung, eine Vorrichtung der eingangs genannten Art so auszubilden, dass sie - bei wirtschaftlich vertretbaren Kosten - großteils wieder verwertbar ist. Dabei soll durch die Wiederverwertbarkeit die Handhabbarkeit in keiner Weise eingeschränkt bzw. verschlechtert werden.

Diese Aufgabe wird ausgehend von einer Vorrichtung der eingangs genannten Art erfindungsgemäß durch die kennzeichnenden Merkmale des Anspruchs 1 gelöst.

Im Rahmen dieser Anmeldung wird dabei der Begriff "proximal" mit der Bedeutung "zum Körper des Arztes hin gelegen" verwendet. Dementspre-chend wird der Begriff "distal" mit der Bedeutung "vom Körper des Arztes entfernt gelegen" verwendet.

Durch die erfindungsgemäße Ausbildung wird erreicht, dass ein Großteil der Vorrichtung nach einer entsprechenden Sterilisation wieder verwendet werden kann, so dass sowohl in wirtschaftlicher wie auch in ökologischer Hinsicht Vorteile erzielt werden. Dabei wird durch die erfindungsgemäß ausgebildete Kopplungseinrichtung die Bedienung der Vorrichtung in keiner Weise beeinträchtigt. Weiter ist durch die erfindungsgemäße Ausbildung gewährleistet, dass genau diejenigen Teile, die während der Applikation mit Knochenzement in Berührung kommen und damit für eine Wiederverwertung nicht in Frage kommen, auf einfache Weise von den übrigen wieder verwertbaren Teilen abgetrennt werden können. Bei entsprechender erfindungsgemäßer Ausgestaltung, kommen die den Knochenzement enthaltende Kammer sowie der in der Kammer angeordnete, den Knochenzement aus der Kammer herauspressende Kolben mit dem Knochenzement in Berührung. Alle weiteren Teile sind bei der erfindungsgemäßen Ausgestaltung so von diesen Elementen entkoppelt, dass sie nicht mit Knochenzement in Berührung kommen und daher wieder verwertet werden können.

In einer vorteilhaften Ausführungsform der Erfindung ist in der Kopplungsstellung der Schaft gegenüber dem Kolben verdrehbar. Dadurch wird erreicht, dass der Kolben innerhalb der Kammer ohne Verdrehung ausschließlich in Längsrichtung geführt wird, wenn beispielsweise der Schaft durch eine Drehbetätigung, wie es weiter unten beschrieben wird, zur Erzeugung des hohen erforderlichen Druckes in Längsrichtung verschoben wird.

Bevorzugt sind in der Kopplungsstellung Kolben und Schaft im Wesentlichen spielfrei in Verschieberichtung miteinander verkoppelt. Durch diese spielfreie Verkopplung ist eine besonders gute Steuerung des austretenden Knochenzements beim Applizieren möglich. Gleichzeitig kann beim Aufziehen des Knochenzements während der Vorbereitung der Vorrichtung ebenfalls eine gute Reaktion erreicht werden.

Bei einer weiteren vorteilhaften Ausführungsform der Erfindung umfasst der Kolben ein insbesondere elastisches Dichtungselement zur Abdichtung der Kolbenaußenseite gegenüber der Innenwand der Kammer vorgesehen. Durch dieses Dichtungselement ist sichergestellt, dass ausschließlich der Kolben und die Kammer mit dem Knochenzement in Berührung kommen. Weiterhin wird durch diese Abdichtung der erforderliche hohe Druck in der Kammer zur Applizierung des Knochenzements sicher aufgebaut.

Bevorzugt können der Kolben und das Dichtungselement als getrennte Teile ausgebildet sein. Auf diese Weise ist für beide Teile jeweils eine optimale Wahl des verwendeten Materials für das Dichtungselement und den Kolben möglich. Grundsätzlich ist es jedoch auch möglich, dass der Kolben und das Dichtungselement integral miteinander ausgebildet sind.

Weiterhin ist der Kolben zumindest in seinem mit der Kopplungseinrichtung zusammenwirkenden Abschnitt starr ausgebildet. Auf diese Weise wird erreicht, dass die Kopplung zwischen der Kopplungseinrichtung und dem Kolben ebenfalls eine starre und unnachgiebige Kopplung ist.

Nach einer weiteren vorteilhaften Ausführungsform der Erfindung ist die Kopplung zwischen dem Kolben und dem Schaft durch eine oder mehrere in Verschieberichtung wirkende Hinterschneidungen gebildet. Diese Hinterschneidungen bilden eine sehr einfache Kopplung zwischen dem Kolben und dem Schaft, wobei bevorzugt sowohl in Aufziehrichtung' als auch in Applikationsrichtung entsprechende Hinterschneidungen wirksam sind, so dass sowohl beim Aufziehen von Knochenzement als auch beim Applizieren von Knochenzement eine sichere Kopplung zwischen Kolben und Schaft besteht.

Nach einer weiteren bevorzugten Ausführungsform der Erfindung sind zur Bildung der Kopplungseinrichtung an dem distalen Ende des Schafts Spreizklauen ausgebildet, wobei an dem proximalen Ende des Kolbens zumindest eine Kopplungsöffnung vorgesehen ist und die Spreizklauen in die Kopplungsöffnung einführbar sind und zur Kopplung mit dem Kolben auseinanderspreizbar oder zusammenziehbar sind. Dabei können bevorzugt die auseinandergespreizte Stellung der Spreizklauen der Kopplungsstellung der Kopplungseinrichtung und die zusammengezogene Stellung der Freigabestellung entsprechen. Grundsätzlich ist es jedoch auch möglich, dass die auseinander gespreizte Stellung der Freigabestellung und die zusammengezogene Stellung der Kopplungsstellung entspricht. In diesem Fall muss hier die Kopplungsöffnung im Kolben beispielsweise als ringförmige Öffnung ausgebildet sein, in deren Mittelpunkt eine von den Spreizklauen hintergreifbare Angriffsfläche vorgesehen ist.

Nach einer weiteren vorteilhaften Ausführungsform der Erfindung ist am proximalen Ende des Schafts ein Betätigungsorgan vorgesehen, über das die Kopplungseinrichtung zwischen der Freigabestellung und der Kopplungsstellung verstellbar ist. Da das proximale Ende des Schafts dem Benutzer üblicherweise zugewandt liegt, ist über dieses Betätigungsorgan eine gute Steuerung der Kopplungseinrichtung sowie eine leichte Zugänglichkeit der Steuerung möglich.

Bevorzugt weist der Schaft eine Längsbohrung auf, durch die ein über das Betätigungsorgan steuerbares Kraftübertragungselement geführt ist, über das eine Betätigung des Betätigungsorgans, insbesondere eine Verdrehung des Betätigungsorgans, in eine Verstellung der Kopplungseinheit umgesetzt wird. Durch diese Ausgestaltung ist in einfacher Weise gewährleistet, dass auch nach Befestigen der Kammer an dem Gehäuse eine einfache Kopplung von Kolben und Schaft möglich ist, obwohl der Kolben sowie die Kopplungseinrichtung zu diesem Zeitpunkt von außen nicht zugänglich innerhalb der Kammer angeordnet sind.

Bevorzugt ist am proximalen Ende des Schafts eine Handhabe zur Längsverschiebung des Schafts vorgesehen. Diese Handhabe kann insbesondere zum drehbaren Verschieben des Schafts wie auch zum direkten Längsverschieben des Schafts ausgebildet sein.

Die Kammer kann vorteilhaft zylinderförmig, insbesondere kreiszylinderförmig ausgebildet sein und kann beispielsweise die Form eines Injektionszylinders besitzen.

Nach einer weiteren vorteilhaften Ausführungsform der Erfindung ist die Kammer drehfest an dem Gehäuse befestigbar. Durch die drehfeste Befestigung ist sichergestellt, dass auch beim Verschieben des Schafts durch eine Drehbewegung entsprechende sich auf die Kammerwand übertragende Rotationskräfte nicht zu einer Drehung der Kammer führen. Da die Kammer üblicherweise über einen Applikationsschlauch mit einer Applikationskanüle verbunden ist, würde eine Rotation der Kammer zu einer entsprechenden Torsion des Applikationsschlauchs führen, was durch die erfindungsgemäße Ausbildung zuverlässig verhindert wird.

Vorteilhaft weist die Befestigungseinrichtung eine Aufnahme auf, in die das insbesondere komplementär zu der Aufnahme ausgebildete proximale Ende der Kammer einsetzbar ist, so dass bei eingesetzter Kammer sowohl eine in Längsrichtung der Verschiebung als auch eine konzentrisch zur Verschieberichtung wirkende kraftschlüssige Verbindung zwischen der Kammer und dem Gehäuse besteht. Durch die sowohl in Längsrichtung als auch in konzentrischer Richtung wirkende kraftschlüssige Verbindung wird sowohl eine Relativbewegung der Kammer bezüglich des Gehäuses in Längsrichtung als auch eine Verdrehung der Kammer bei der Applikation des Knochenzements zuverlässig verhindert.

Insbesondere kann das proximale Ende der Kammer einen Ansatz aufweisen, der in die Aufnahme einsetzbar ist. Dieser Ansatz kann beispielsweise als umlaufender Falz ausgebildet sein, der bereichsweise Abflachungen bzw. von der konzentrischen Form abweichende Ausnehmungen oder Ausbuchtungen besitzt, durch die eine Verdrehung der Kammer in Zusammenwirkung mit entsprechenden Formen der Aufnahme verhindert werden.

Vorteilhaft kann der Schaft durch eine Schraubbewegung innerhalb des Gehäuses längsverschiebbar sein, so dass aufgrund der Schraubbewegung ein hoher Druck bei der Applikation des Knochenzements innerhalb der Kammer erzeugbar ist. Bevorzugt ist die Vorrichtung zwischen der Verschiebung des Schafts durch die Schraubbewegung und einer direkten Verschiebung in Längsrichtung ohne Schraubbewegung umstellbar, wodurch insbesondere beim Aufziehen des Knochenzements bzw. beim Beginn der Applikation zunächst durch die direkte Verschiebung der Schaft auf schnelle Weise bewegt werden kann. Erst nachdem beim Applizieren der erforderliche Druck so groß wird, dass mit der direkten Verschiebung eine weitere Bewegung des Schafts nicht mehr möglich ist, wird auf die Verschiebung durch Schraubbewegung umgestellt, wodurch ein wesentlich höherer Druck erzeugt werden kann.

Nach einer weiteren vorteilhaften Ausführungsform der Erfindung umfasst der Schaft einen Eingriffsabschnitt mit einem Schraubgewinde, das in eine am Gehäuse vorgesehene Gegenverzahnung eingreift, so dass beim Verdrehen des Schafts die Längsverschiebung des Kolbens erfolgt. Auf diese Weise wird ein sehr einfacher, kostengünstiger und funktionssicherer Aufbau einer erfindungsgemäßen Applikationsvorrichtung erreicht. Insbesondere wird in dem Betriebsmodus "Verschiebung durch Schraubbewegung" durch die ineinander greifenden Verzahnungen automatisch eine direkte Verschiebung in Längsrichtung verhindert, so dass die mit jeder Umdrehung erzielte Erhöhung des angewendeten Drucks automatisch gesichert ist.

Nach einer weiteren bevorzugten Ausführungsform der Erfindung sind das Schraubgewinde und die Gegenverzahnung voneinander lösbar. Dabei kann die Gegenverzahnung vorteilhaft zum Lösen in einer Richtung im Wesentlichen senkrecht zur Verschieberichtung des Kolbens bewegbar sein. Durch diese Ausbildung ist eine einfache und schnelle Umschaltung von dem Betriebszustand "Verschieben durch Schraubbewegung" in den Betriebszustand "Direkte Verschiebung in Längsrichtung und zurück" möglich, indem beispielsweise über einen am Gehäuse angebrachten Betätigungsabschnitt die Gegenverzahnung von dem Schraubgewinde entkoppelt wird.

Vorteilhaft wird die Gegenverzahnung unter Vorspannung gegen das Schraubgewinde gedrückt. Dadurch ist gewährleistet, dass der aufgebaute Druck so lange automatisch gesichert ist, bis die Gegenverzahnung entgegen der Vorspannung bewegt wird. Diese Vorspannung kann beispielsweise durch eine Federbeaufschlagung erfolgen.

Bevorzugt ist im gelösten Zustand der Schaft im Wesentlichen frei im Gehäuse längsverschiebbar. Die freie Verschiebbarkeit des Schafts ist dabei im Wesentlichen nur durch eine Dichtung eingeschränkt, die üblicherweise zwischen dem Kolbenumfang und der Innenwand der Kammer zur Abdichtung vorgesehen ist.

Nach einer weiteren vorteilhaften Ausführungsform der Erfindung ist in dem Gehäuse ein Sperrelement verschiebbar gelagert, wobei das Sperrelement eine Durchgangsöffnung besitzt, durch die der Schaft geführt ist, innerhalb der Durchgangsöffnung die Gegenverzahnung ausgebildet ist und bei einem Verschieben des Sperrelements in einer Richtung das Schraubgewinde mit der Gegenverzahnung in Eingriff kommt, während bei einer Verschiebung das Sperrelement in der entgegengesetzten Richtung das Schraubgewinde und die Gegenverzahnung außer Eingriff kommen und der Schaft innerhalb der Durchgangsöffnung frei längsverschiebbar ist. Bevorzugt ist dabei das Sperrelement zum Verschieben von außerhalb des Gehäuses zugreifbar.

Durch diese Ausführung ist eine sehr kostengünstige Ausbildung der Verzahnung zwischen Schraubgewinde und Gegenverzahnung möglich, indem lediglich ein einziges Sperrelement, das unter Federvorspannung steht, vorgesehen ist. Das Sperrelement kann in dem Fall gleichzeitig den Betätigungsabschnitt bilden, so dass keine weiteren Elemente für die Umschaltung zwischen direkter Verschiebung und Verschiebung durch Drehbewegung erforderlich ist.

Dazu kann beispielsweise der der Gegenverzahnung gegenüberliegende Bereich des Sperrelements einen von außerhalb des Gehäuses zugreifbaren Betätigungsabschnitt bilden, wobei bei Drücken auf den Betätigungsabschnitt das Schraubgewinde und die Gegenverzahnung außer Eingriff geraten. Die Betätigung erfolgt dabei gegen die Vorspannung des Sperrelements, so dass nach Freigabe des Sperrelements automatisch die Gegenverzahnung wieder in das Schraubgewinde eingreift und die Vorrichtung automatisch wieder in den Modus "Verschieben durch Schraubbewegung" überführt wird.

Nach einer weiteren vorteilhaften Ausführungsform der Erfindung schließen die beim Applizieren des Knochenzements unter Druck aneinander anliegenden Zahnflanken der Gegenverzahnung und/ oder der Flanken des Gewindeprofils des Schraubgewindes einen Winkel von kleiner oder gleich ca. 90° mit der zur Verschieberichtung parallel verlaufenden Längsachse des Schafts ein. Durch diese spezielle Ausbildung der Flanken ist gewährleistet, dass auch bei Anwendung sehr hoher Drücke kein Überschnappen einzelner Zähne auftritt, wie es beispielsweise bei üblichen, abschrägten Flanken der Fall sein kann, bei denen der Winkel zwischen den Flanken und der Längsachsen des Eingriffsabschnitts größer als 90° ist. Beträgt der Winkel im Wesentlichen gleich 90°, so kann die Gegenverzahnung zum Entkoppeln von dem Schraubgewinde durch eine Schiebebewegung senkrecht zur Bewegungsrichtung des Eingriffsabschnitts verschoben werden. Sind die Winkel kleiner als 90° so ist eine Entkopplung durch eine entsprechend Verschiebung der Gegenverzahnung schräg zur Längsachse des Schafts möglich.

Vorteilhaft ist an der Austrittsöffnung der Kammer ein Anschluss für eine Kanüle, insbesondere eine Luer-Lock-Verbindung vorgesehen. Über diesen Anschluss kann entweder eine Kanüle direkt oder beispielsweise über einen Hochdruckschlauch befestigt werden.

Vorteilhaft sind alle während der Applikation mit Knochenzement in Berührung kommende Teile als Wegwerfartikel und insbesondere aus Kunststoff ausgebildet. Die während der Applikation nicht mit Knochenzement in Berührung kommenden Teile der Vorrichtung sind hingegen als mehrmals verwendbare Artikel, insbesondere großteils aus Metall für eine dauerhafte Verwendung ausgebildet. Dies betrifft insbesondere das Gehäuse und/oder den Schaft, während insbesondere die Kammer und/oder der Kolben aus Kunststoff bestehen können.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: eine Seitenansicht einer erfindungsgemäß ausgebildeten Applikationsvorrichtung ohne Applikationskammer und Applikationskolben,
- Fig. 2: eine weitere Seitenansicht auf die Vorrichtung nach Fig. 1 mit gegenüber Fig. 1 in proximaler Richtung verschobenem Schaft und teilweise dargestellter, nicht eingesetzter Applikationskammer,
- Fig. 3: einen Ausschnitt der Vorrichtung nach Fig. 2 mit eingesetzter Applikationskammer,
- Fig. 4: einen Ausschnitt der Vorrichtung nach Fig. 2 mit teilweise geschnitten dargestellter, geöffneter Befestigungseinrichtung und noch nicht eingesetzter Applikationskammer,
- Fig. 5: eine Vorderansicht auf die Vorrichtung nach Fig. 4 mit geöffneter Befestigungseinrichtung,
- Fig. 6: eine Seitenansicht einer Applikationskammer, wie sie in den Fig. 2 bis 4 teilweise dargestellt ist,
- Fig. 7: eine weitere Seitenansicht der Applikationskammer nach Fig. 6,
- Fig. 8: eine Rückansicht der Applikationskammer nach Fig. 6 und 7,
- Fig. 9: einen Teilquerschnitt durch die Vorrichtung nach Fig. 2 entlang der Linie A-A mit verriegeltem Schaft,
- Fig. 10: die Darstellung nach Fig. 9 mit entriegeltem Schaft,
- Fig. 11: einen Längsschnitt durch einen Schaft einer erfindungsgemäß ausgebildeten Vorrichtung und
- Fig. 12: einen teilweise aufgerissenen Längsschnitt durch eine erfindungsgemäß ausgebildete Vorrichtung mit befestigter Applikationskammer und darin angeordnetem Schaft.

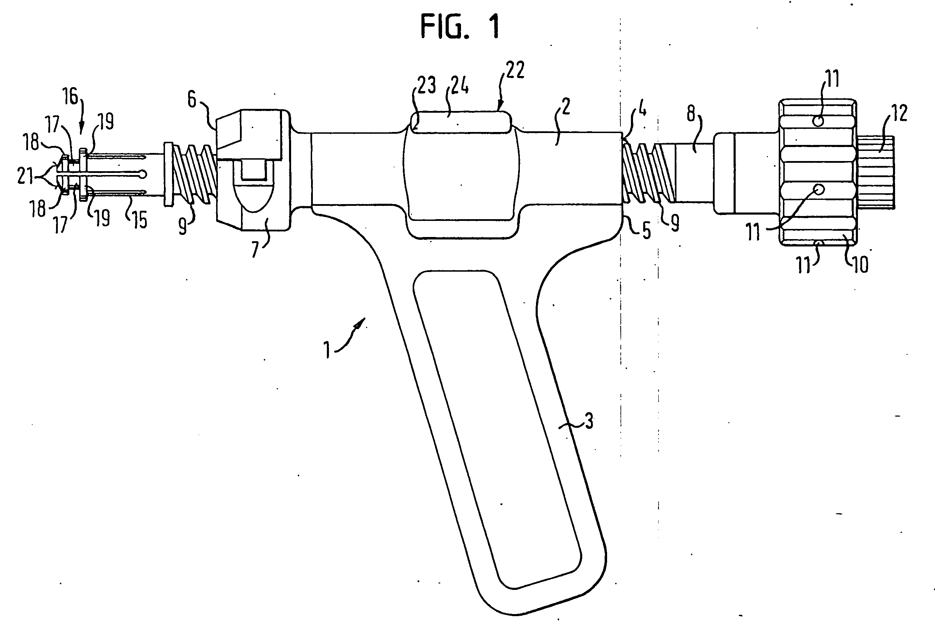

Fig. 1 zeigt eine pistolenförmige Applikationsvorrichtung 1 mit einem Gehäuse 2, das an seiner Unterseite einen Handgriff 3 besitzt. Der obere Bereich des Gehäuses 2 besitzt eine zylinderförmige Durchgangsöffnung 4, die sich vom in Fig. 1 rechts liegenden proximalen Ende 5 bis zu dem gegenüberliegenden distalen Ende 6 des Gehäuses 2 erstreckt. An dem distalen Ende 6 ist eine Befestigungseinrichtung 7 vorgesehen, die unter Bezugnahme auf die weiteren Figuren nachfolgend näher beschrieben wird.

Innerhalb des Gehäuses 2 ist ein sich durch die Durchgangsöffnung 4 erstreckender, einen konzentrischen Querschnitt besitzender Schaft 8 angeordnet, der in seinem mittleren Bereich mit einem außen liegenden Schraubgewinde 9 versehen ist.

An dem proximalen Ende des Schafts 8 ist eine Handhabe 10 ausgebildet, die durch eine zylinderförmige Verdickung des proximalen Endes des Schafts 8 gebildet wird. Die Umfangsfläche der Handhabe 8 ist dabei mit Riffelungen versehen, um eine gute Griffigkeit für ein Verdrehen des Schafts über die Handhabe 10 zu erreichen.

Weiterhin sind in der Handhabe 10 Spülöffnungen 11 ausgebildet, die ebenfalls unter Bezugnahme auf die Figuren später erläutert werden.

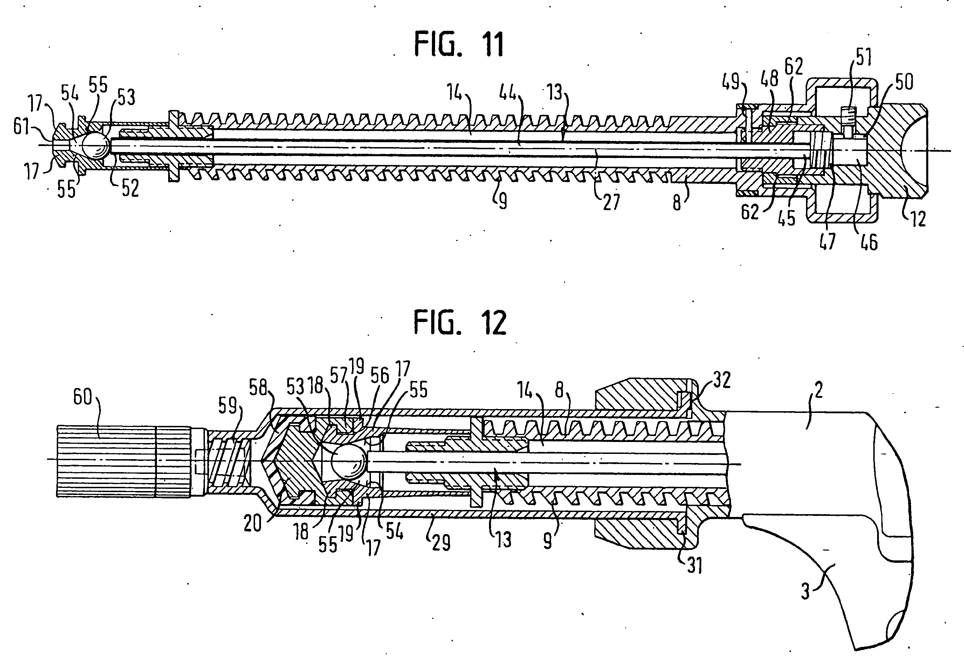

An die Handhabe 10 schließt sich proximalseitig ein als Drehknopf augebildetes Betätigungsorgan 12 an, über das eine im Inneren des Schafts 8 angeordnete, in Fig. 1 nicht sichtbare Kraftübertragungseinheit 13 (siehe Fig. 11 und 12) betätigbar ist. Die Kraftübertragungseinheit 13 erstreckt sich durch einen über die gesamte Länge des Schafts verlaufenden Hohlraum 14 (siehe Fig. 11 und 12) bis hin zum distalen Ende 15 des Schafts 8.

Das distale Ende 15 des Schafts 8 ist als Kopplungseinrichtung 16 ausgebildet, die eine Vielzahl von Spreizklauen 17 umfasst. Die Spreizklauen 17 umfassen dabei jeweils zwei als Ansätze ausgebildete Anschläge 18, 19, die jeweils für einen in Fig. 1 nicht dargestellten Kolben 20 (Fig. 12) Hinterschneidungen bilden.

Weiterhin ist die distale Stirnseite des Schafts 8 mit Auflaufschrägen 21 versehen.

In der Mitte des Gehäuses 2 ist ein Sperrelement 22 ausgebildet, das in eine in der Oberseite des Gehäuses 2 ausgebildete Öffnung 23 eingesetzt ist. Der obere Bereich des Sperrelements 22 bildet einen aus dem Gehäuse 2 herausragenden Betätigungsabschnitt 24, wobei das Sperrelement 22 so in der Öffnung 23 des Gehäuses 2 verschiebbar gelagert ist, dass durch Drücken auf den Betätigungsabschnitt 24 das Sperrelement 22 tiefer in das Gehäuse 2 hinein verschoben wird.

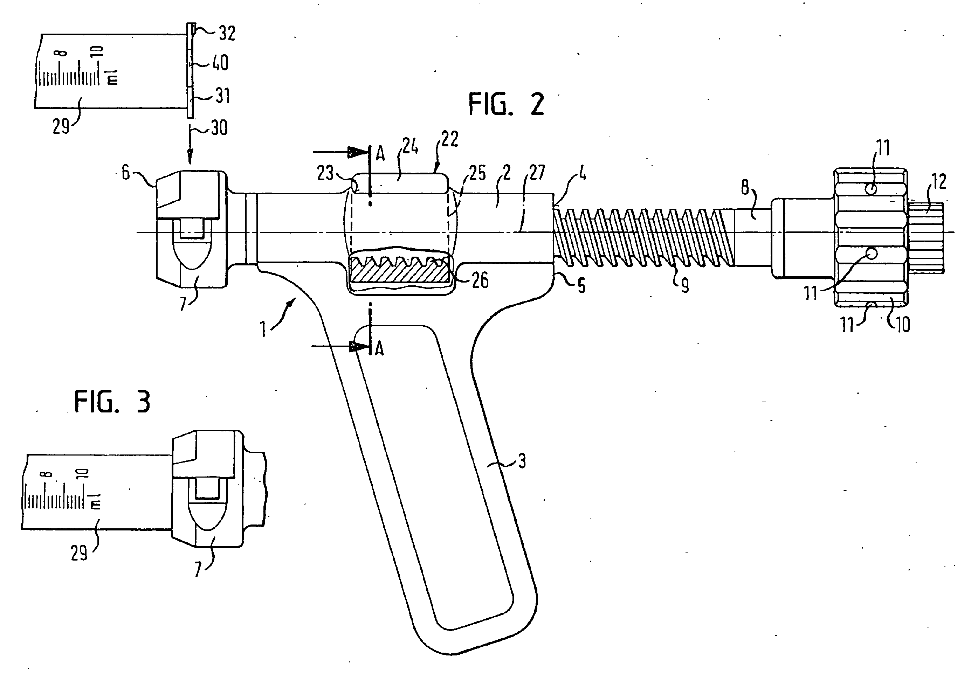

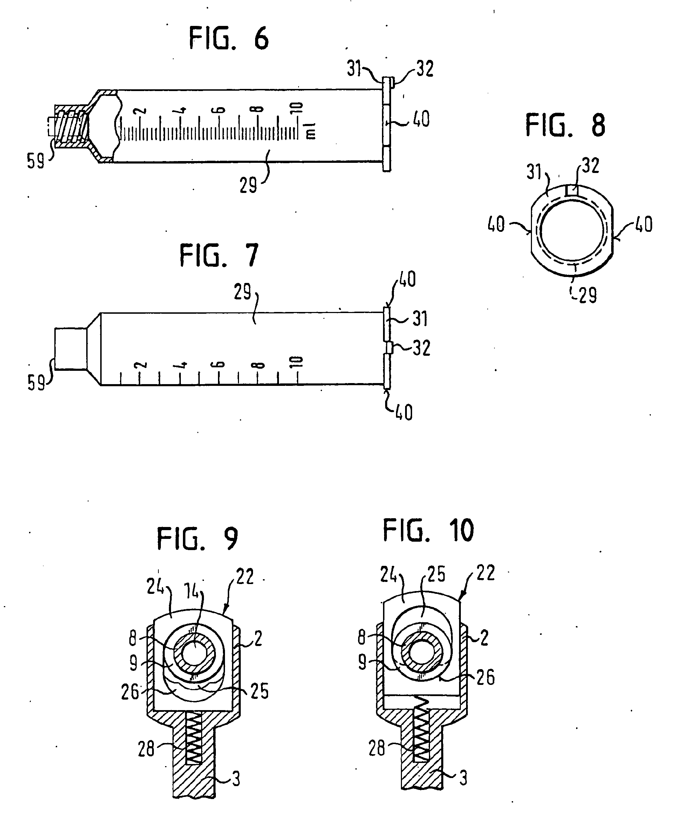

Wie aus der teilweise aufgerissenen Darstellung nach Fig. 2 zu erkennen ist, besitzt das Sperrelement 22 eine Durchgangsöffnung 25, durch die der Schaft 8 hindurchgeführt ist. An der Unterseite der Innenwand der Durchgangsöffnung 25 ist eine Gegenverzahnung 26 ausgebildet, die in das Schraubgewinde 9 des Schafts 8 eingreift und in der in Fig. 2 dargestellten Stellung ein direktes Verschieben des Schafts 8 entlang der Längsachse 27 verhindert. Wie insbesondere auch aus den Fig. 9 und 10 zu erkennen ist, besitzt die Durchgangsöffnung 25 in senkrechter Richtung eine lichte Weite, die größer ist als der Außendurchmesser des Schraubgewindes 9 des Schafts 8. Weiterhin ist aus den Fig. 9 und 10 zu erkennen, dass das Sperrelement 22 an seiner Unterseite von einer Feder 28 beaufschlagt wird und durch diese Feder 28 nach oben gedrängt wird, wie es in Fig. 10 dargestellt ist. In dieser Position ist die Gegenverzahnung 26 mit dem Schraubgewinde 9 in Eingriff, so dass ein Verschieben des Schafts 8 verhindert wird.

Durch Krafteinwirkung auf den Betätigungsabschnitt 24 des Sperrelements 22 wird dieses nach unten gegen die Federvorspannung der Feder 28 verschoben, so dass die Gegenverzahnung außer Eingriff mit dem Schraubgewinde 9 des Schafts 8 gerät, wodurch der Schaft 8 innerhalb der Durchgangsöffnung 25 des Sperrelements 22 und damit innerhalb der Durchgangsöffnung 4 des Gehäuses 2 im Wesentlichen frei verschiebbar ist.

Sind die Gegenverzahnung 26 und das Schraubgewinde 9 des Schafts 8 in Eingriff, wie es in Fig. 10 dargestellt ist, so ist eine Verschiebung des Schafts 8 entlang der Längsachse 27 durch eine Schraubbewegung möglich, wobei die Schraubbewegung insbesondere über die Handhabe 10 ausgeführt werden kann.

In Fig. 2 ist weiterhin angedeutet, dass in die Befestigungseinrichtung 7 ein eine Applikationskammer bildender Applikationszylinder 29 gemäß einem Pfeil 30 einsetzbar ist. Der Applikationszylinder 29 besitzt zur Festlegung in der Befestigungseinrichtung 7 an seinem proximalen Ende einen umlaufenden Ansatz 31 mit einer sich daran anschließenden Nase 32, die unter Bezugnahme auf die weiteren Figuren nachfolgend näher beschrieben werden.

Fig. 3 zeigt den in die Befestigungseinrichtung 7 eingesetzten Applikationszylinder 29, der durch die Befestigungseinrichtung 7 sowohl in Längsrichtung (entlang der Längsachse 27) unverschiebbar als auch verdrehfest mit dem Gehäuse 2 verbunden ist.

Der Schaft 8 ist in Fig. 2 so weit in proximaler Richtung zurückgezogen, dass sein distales Ende mit der Befestigungseinrichtung 7 vollständig innerhalb des Gehäuses 2 zu liegen kommt und in Fig. 2 nicht zu sehen ist.

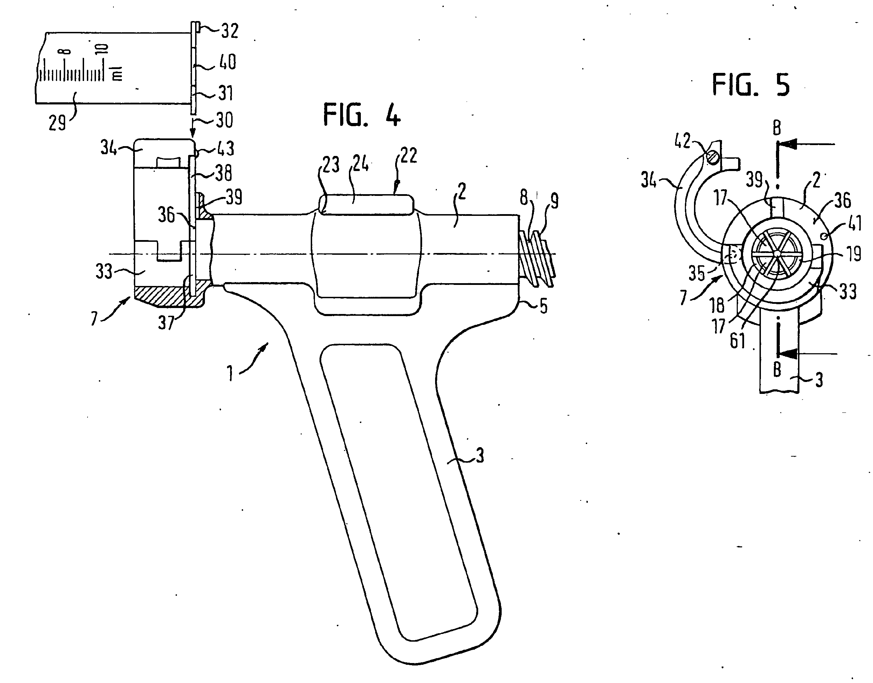

Aus den Fig. 4 und 5 ist die Befestigungseinrichtung 7 in ihrer geöffneten Stellung zu erkennen.

Die Befestigungseinrichtung 7 umfasst eine U-förmige Aufnahme 33, die als Fortführung des Kreises 2 integral mit diesem ausgebildet ist. Weiterhin ist eine U-förmige Klammer 34 vorgesehen, die über eine Schwenkachse 35 verschwenkbar an der U-förmigen Aufnahme 33 befestigt ist.

In der Aufnahme 33 ist unmittelbar im Bereich der Stirnfläche 36 des Gehäuses 2 eine Aufnahmenut 37 ausgebildet, in die bei eingesetztem Applikationszylinder 29 der Ansatz 31 zu liegen kommt.

Entsprechend ist in der U-förmigen Klammer 34 ebenfalls eine Aufnahmenut 38 ausgebildet, in die nach Verschwenken der U-förmigen Klammer 34 um die Schwenkachse 35 der obere Bereich des Ansatzes 31 des Applikationszylinders 29 zu liegen kommt. Weiterhin ist aus Fig. 4 eine in der Stirnfläche 36 des Gehäuses 2 ausgebildete Zentriernut 39 zu erkennen, in der bei eingesetztem Applikationszylinder 29 die Nase 32 zu liegen kommt. Durch diese Nase 32 wird somit neben der Zentriereigenschaft auch eine Verdrehsicherheit des Applikationszylinders 29 erreicht.

Eine weitere Verdrehsicherheit wird dadurch erreicht, dass der Ansatz 31 des Applikationszylinders 29 an zwei gegenüberliegenden Seiten Abflachungen 40 besitzt, die aus den Fig. 6 bis 8 zu erkennen sind und mit entsprechenden Gegenelementen an der Aufnahme 33 und der Klammer 34 zusammenwirken.

In Fig. 5 ist weiterhin eine in der Stirnfläche 36 ausgebildete Rastöffnung 41 zu erkennen, die mit einer in der U-förmigen Klammer 34 vorgesehenen, durch eine Stellschraube 42 gegen eine Federvorspannung in üblicher Weise gesicherte Rastkugel 43 zum Einrasten der U-förmigen Klammer 34 vorgesehen ist.

Aus dem in Fig. 11 dargestellten Längsschnitt durch den Schaft 8 sind der über die gesamte Länge des Schafts verlaufende Hohlraum 14 sowie die darin angeordnete Kraftübertragungseinheit 13 zu erkennen. Die Kraftübertragungseinheit 13 umfasst eine Druckstange 44, deren proximales Ende 45 von einem Druckelement 46 beaufschlagt wird. Das Druckelement 46 besitzt einen Gewindeabschnitt 47, über den das Druckelement 46 mit einer ein Innengewinde umfassende Gewindebuchse 48 verschraubt ist. Die Gewindebuchse 48 ist über ein Befestigungselement 49, beispielsweise eine Madenschraube, unverdrehbar mit dem Schaft 9 verbunden.

In der in Fig. 11 rechts liegenden Hälfte des Druckelements 46 ist eine sich parallel zur Längsachse 27 erstreckende Führungsnut 50 ausgebildet, in die eine einen Mitnehmer bildende, sich radial durch das Betätigungsorgan 12 hindurch erstreckende Schraube 51 eingreift.

Beim Verdrehen des Betätigungsorgans 12 wird über die Schraube 51 auch das Druckelement 46 verdreht, so dass sich dieses mit seinem Gewindeabschnitt 47 entlang der Längsachse 27 in die Gewindebuchse 48 einschraubt. Auf diese Weise wird somit bei einer Drehung des Betätigungsorgans 12 eine entlang der Längsachse 27 in distaler Richtung des Schafts 9 wirkende Kraft auf die Druckstange 44 erzeugt, so dass diese in distaler Richtung verschoben wird.

Das proximale Ende 52 der Druckstange 44 liegt an einer Kugel 53 an, die in einem zwischen den Spreizklauen 17 angeordneten Hohlraum 54 angeordnet ist. Dabei sind die Innenseiten der Spreizklauen 17 mit Auflaufschrägen 45 versehen, an denen die Kugel 53 bei einem Verschieben der Druckstange 44 in distaler Richtung zur Anlage kommt. Wird die Druckstange 44 durch weiteres Verdrehen des Betätigungsorgans 12 weiter in distaler Richtung verschoben, so werden die Spreizklauen 17 durch die an den Auflaufschrägen 45 anlaufende Kugel 53 radial nach außen gespreizt, während bei einem Zurückziehen der Druckstange 44 durch entgegengesetzte Verdrehung des Betätigungsorgans 12 sich die Spreizklauen 17 aufgrund ihrer Federwirkung wieder in die in Fig. 11 dargestellte Ruheposition begeben.

In Fig. 12 ist der auseinander gespreizte Zustand der Spreizklauen 17 dargestellt. Dabei sind die Spreizklauen 17 mit ihren distalen Enden in eine Öffnung 56 des Kolbens 20 eingesetzt, wobei die Öffnung 56 einen umlaufenden Ringwulst 57 besitzt, der in Richtung der Längsachse 27 eine Hinterschneidung für die Anschläge 18, 19 der Spreizklauen 17 bildet.

Wie aus Fig. 12 zu erkennen ist, ist somit bei geöffneten Spreizklauen 17 eine Kopplung zwischen den die Kopplungseinrichtung bildenden Spreizklauen 17 und dem Kolben 20 gegeben, durch die eine Längsverschiebung des Kolbens 20 zusammen mit dem Schaft 8 in beiden Richtungen entlang der Längsachse 27 gewährleistet ist.

Der Kolben 20 ist von einem aus elastischem Material, beispielsweise aus Gummi oder Silikon, bestehenden Dichtung 58 umgeben, durch die eine Abdichtung zwischen dem Kolben 20 und der Innenwand der Applikationskammer 29 erfolgt.

Weiterhin ist aus Fig. 12 zu erkennen, dass die Applikationskammer distalseitig eine Austrittsöffnung 59 besitzt, an der ein Anschluss für eine Kanüle in Form einer Luer-Lock-Verbindung 60 vorgesehen ist.

Eine erfindungsgemäß ausgebildete Vorrichtung wird wie folgt verwendet:

Zur Vorbereitung der Applikationsvorrichtung 1 wird zunächst der Schaft 8 vollständig in die in Fig. 2 gezeigte Stellung zurückgezogen. Dazu wird das Sperrelement 22 durch Drücken auf den Betätigungsabschnitt 24 gegen die Kraft der Federn 28 nach unten verschoben, so dass die Verzahnung 26 außer Eingriff mit dem Schraubgewinde 9 kommt. Der Schaft 8 ist auf diese Weise frei innerhalb der Öffnung 4 des Gehäuses 2 verschiebbar und kann in einfacher Weise direkt bis zu einem Anschlag nach hinten gezogen werden.

In dieser Stellung wird anschließend die Befestigungseinrichtung 7 durch Aufklappen der U-förmigen Klammer 34 um die Schwenkachse 35 geöffnet. Anschließend wird der Applikationszylinder 29 in die Befestigungseinrichtung 7 eingesetzt, so dass der Ansatz 31 in der Aufnahmenut 37 zu liegen kommt. Dabei wird der Aufnahmezylinder 29 so positioniert, dass die Nase 32 in der Zentriernut 39 angeordnet ist.

Zur Befestigung wird daraufhin die U-förmige Klammer 34 in ihre verschlossene Stellung verschwenkt, bis die Rastkugel 43 in der Rastöffnung 41 zur Fixierung der U-förmigen Klammer 34 einrastet. In dieser Stellung ist der oben liegende Bereich des Ansatzes 31 in der Aufnahmenut 38 angeordnet, so dass der Applikationszylinder 29 fest, sowohl in Längsrichtung unverschiebbar als auch unverdrehbar, durch die Befestigungseinrichtung 7 gehalten wird.

In dem Applikationszylinder 29 ist zu diesem Zeitpunkt der Kolben 20 mit der Dichtung 58 angeordnet, die vor Einsetzen des Applikationszylinders 29 aufgrund der Reibung zwischen der Dichtung 58 und der Innenwand des Applikationszylinders 29 vor einem Herausfallen aus dem Applikationszylinder 29 gesichert waren.

Zum Verkoppeln des Schafts 8 mit dem Kolben 20 wird das Sperrelement 22 über den Betätigungsabschnitt 24 nach unten gedrückt, so dass der Schaft 8 frei in proximaler Richtung verschoben werden kann. Das Betätigungsorgan 12 ist dabei so in eine erste Endstellung verdreht, dass das Druckelement 46 vollständig in proximaler Richtung verschoben ist. Die in proximaler Richtung gelegene Endstellung des Druckelements 46 ist dabei durch einen Anschlag definiert. Die Druckstange 44 befindet sich ebenfalls in ihrer vollständig zurückgezogenen, proximalen Stellung, in die sie durch die Kugel 53 aufgrund der Federwirkung der an der Kugel 53 über die Auflaufschrägen 55 anliegenden Spreizklauen 17 gedrängt wird. Die Spreizklauen 17 befinden sich somit in ihrer zusammengezogenen Stellung, so dass die von den Spreizklauen 17 und der Kugel 53 gebildete Kopplungseinrichtung sich in ihrer Freigabestellung befindet.

In dieser Freigabestellung ist der von den distal gelegenen Anschlägen 18 gebildete äußere Durchmesser kleiner als die lichte Weite der Öffnung 56 des Kolbens 20, so dass durch Verschieben des Schafts 8 in distale Richtung die Spreizklauen 17 in die Öffnung 56 des Kolbens 20 eingesetzt werden.

Anschließend wird durch Verdrehen des Betätigungsorgans 12 das Druckelement 46 in distaler Richtung verschoben, wodurch sich auch die an der Stirnseite des Druckelements 46 anliegende Druckstange 44 in distaler Richtung bewegt. Diese Bewegung wird durch den Schaft 8 hindurch auf die Kugel 53 übertragen, die zur Anlage an die Auflaufschrägen 55 gelangt und bei einem weiteren Verschieben ein Auseinanderspreizen der Spreizklauen 17 bewirkt, wie es in Fig. 12 dargestellt ist. Auch diese Verschiebung in distaler Richtung durch das Betätigungsorgan 12 ist durch einen Anschlag begrenzt, so dass das Auseinanderspreizen der Spreizklauen 17 beendet ist, bevor eine Klemmwirkung zwischen den Spreizklauen 17 und dem Kolben 20 erzeugt wird. Der Kolben 20 ist somit zwar in Längsverschiebung durch die Hinterschneidungen zwischen den Ansätzen 18 bzw. 19 der Spreizklauen 17 und dem umlaufenden Ringwulst 57 des Kolbens 20 gesichert, bleibt jedoch gegenüber dem Schaft 8 verdrehbar. Selbst wenn das Auseinanderspreizen der Spreizklauen 17 dazu führen würde, dass die Spreizklauen 17 mit dem Kolben 20 in Klemmwirkung geraten, so ist dies unproblematisch, da der Kolben 20 aus einem starren Material, beispielsweise aus Hartplastik oder aus Metall ausgebildet ist und somit kein Verklemmen des Kolbens 20 innerhalb des Applikationszylinders 29 erfolgen kann. Die auf dem Kolben aufgesetzte, aus elastischem Material bestehende Dichtung 58 bleibt von dieser Spreizwirkung unberührt.

Nach dieser Verkopplung des Schafts 8 mit dem Kolben 20 wird der angerührte Knochenzement über eine mit der Luer-Lock-Verbindung 60 verbundene Aufziehkanüle in das Innere des Applikationszylinders 29 aufgezogen. Dazu wird das Sperrelement 22 entgegen der Kraft der Federn 28 über den Betätigungsabschnitt 24 so nach unten gedrückt, dass die Verzahnung 26 außer Eingriff mit dem Schraubgewinde 9 kommt. Auf diese Weise kann durch einfaches Zurückziehen des Schafts 8 zusammen mit dem Kolben 22 der Knochenzement über die Aufziehkanüle in das Innere des Applikationszylinders 29 gezogen werden.

Anschließend wird die Aufziehkanüle von der Applikationsvorrichtung 1 getrennt und diese mit einer bereits in Position gebrachten Injektionskanüle verbunden. Zu diesem Zeitpunkt befindet sich der Schaft 8 in seiner äußersten zurückgezogenen Stellung, wie sie in Fig. 2 dargestellt ist. Im nächsten Verfahrensschritt wird wiederum bei gelöstem Sperrelement 22 der Kolben 20 mit dem Schaft 8 durch direktes Verschieben des Schafts 8 über die Handhabe 10 bzw. das Betätigungsorgan 12 direkt in den Applikationszylinder 29 hinein verschoben, wodurch das im Inneren des Applikationszylinders 29 angeordnete Zementmaterial über die angeschlossene Kanüle in den Knochen injiziert wird. Durch die Injektion in das Knochenmaterial wird ein ständig wachsender Druck aufgebaut, bis dieser schließlich so hoch wird, dass ein weiteres Applizieren des Knochenzements durch direktes Vorschieben des Schafts 8 nicht mehr möglich ist.

Zu diesem Zeitpunkt wird das Sperrelement 22 freigegeben, so dass dieses durch die Kraft der Federn 28 nach oben verschoben wird, bis die Gegenverzahnung 26 mit dem Schraubgewinde 9 in Eingriff ist und ein direktes Verschieben des Schafts 8 in distaler und proximaler Richtung entlang der Längsachse 27 blockiert wird.

Anschließend kann durch Verdrehen des Schafts 8 über die Handhabe 10 der Druck im Inneren des Applikationszylinders 29 weiter erhöht werden, so dass der Kolben 20 weiter langsam innerhalb des Applikationszylinders 29 in distaler Richtung verschoben wird.

Ist die aus der Öffnung 59 des Applikationszylinders 29 austretende Menge des Knochenzements aufgrund des hohen Drucks zu groß, so kann dieser Druck unverzüglich durch Verschieben des Sperrelements 22 und die dadurch erfolge Entkopplung und Freigabe des Schafts 9 und des damit verbundenen Kolbens 20 abgebaut werden. Auf diese Weise wird verhindert, dass der Knochenzement an gefährliche Stellen innerhalb des Körpers appliziert wird.

Nach erfolgter Applikation des Knochenzements kann die Applikationsvorrichtung 1 von der Applikationskanäle getrennt werden.

Anschließend wird durch Verdrehen des Betätigungsorgans 12 in entgegengesetzter Richtung das Druckelement 46 in proximaler Richtung verschoben, so dass auch die an der Stirnseite des Druckelements 46 anliegende Druckstange 44 aufgrund der über die Kugel 53 und die Auflaufschrägen 55 durch die Federwirkung der Spreizklauen 17 wirkenden Kraft in proximaler Richtung verschoben wird, bis sich die Spreizklauen 17 wieder in ihrer zusammengezogenen Freigabestellung befinden, die in Fig. 11 gezeigt ist.

In dieser Stellung ist der von den Anschlägen 18 gebildete Außendurchmesser kleiner als die lichte Weite der Öffnung 56 des Kolbens 20, so dass durch Zurückziehen des Schafts 8, entweder nach Lösen des Sperrelements 22 oder durch Verdrehen über die Handhabe 10, der Schaft 8 von dem Kolben 8 entkoppelt werden kann. Der Schaft 8 wird dabei bis in seine in Fig. 2 dargestellte proximale Endposition zurückgezogen, woraufhin die Befestigungseinrichtung 7 durch Aufklappen der U-förmigen Klammer 34 geöffnet und der Applikationszylinder 29 aus der Befestigungseinrichtung 7 entnommen werden kann.

Für eine einfache Bedienung ist es dabei vorteilhaft, wenn die beim Verdrehen des Betätigungsorgans 12 auftretenden Reibungskräfte deutlich geringer sind, als die durch die Federn 28 erzeugten Reibungskräfte zwischen der Gegenverzahnung 26 und dem Schraubgewinde 9. Wenn das Sperrelement 22 über seine Gegenverzahnung 26 mit dem Schraubgewinde 9 in Eingriff steht, wird auf diese Weise erreicht, dass ein Verdrehen des Betätigungsorgans 12 und damit sowohl ein Überführen der Kopplungseinrichtung 16 in ihre Freigabestellung als auch in ihre Kopplungsstellung möglich ist, ohne dass die Handhabe 10 mit der anderen Hand gleichzeitig festgehalten werden muss. Der Wechsel zwischen der Freigabestellung und der Kopplungsstellung der Kopplungseinrichtung ist somit durch eine Einhandbedienung möglich.

Nachdem während der Applizierung des Knochenzements ausschließlich der Injektionszylinder 29 sowie die Dichtung 58 und, je nach Ausbildung, eventuell der separat ausgebildete Kolben 20 mit dem Knochenzement in Kontakt kommen, werden diese Elemente nach der erfolgten Applikation entsorgt.

Alle übrigen Elemente der Applikationsvorrichtung 1 können anschließend in einem Rückspülvorgang gesäubert und danach sterilisiert werden.

Für die Rückspülung wird entsprechende Reinigungsflüssigkeit unter hohem Druck durch die distalseitig zwischen den Spreizklauen 17 ausgebildete Öffnung 61 eingebracht, die sich durch den sich über den gesamten Schaft 8 erstreckenden Hohlraum 14 verteilt. Am Ende des Hohlraums 14 sind im Bereich der Außenseite der Gewindebuchse 48 Rückspülkanäle 62 ausgebildet, über die die Reinigungsflüssigkeit in das Innere der Handhabe 10 geleitet wird, so dass sie anschließend über die in der Handhabe 10 ausgebildeten Spülöffnungen 11 austreten kann.

Auf diese Weise ist eine einfache und vollständige Reinigung der wieder verwendbaren Teile der Applikationsvorrichtung 1 möglich.

### Bezugszeichenliste

- 1: Applikationsvorrichtung
- 2: Gehäuse
- 3: Handgriff
- 4: Durchgangsöffnung
- 5: proximales Ende des Gehäuses
- 6: distales Ende des Gehäuses
- 7: Befestigungseinrichtung
- 8: Schaft
- 9: Schraubgewinde
- 10: Handhabe
- 11: Spülöffnungen
- 12: Betätigungsorgan
- 13: Kraftübertragungseinheit
- 14: Hohlraum
- 15: distales Ende des Schafts
- 16: Kopplungseinrichtung
- 17: Spreizklauen
- 18: Anschläge
- 19: Anschläge
- 20: Kolben
- 21: Auflaufschrägen
- 22: Sperrelement
- 23: Öffnung
- 24: Betätigungsabschnitt
- 25: Durchgangsöffnung
- 26: Gegenverzahnung
- 27: Längsachse
- 28: Federn
- 29: Applikationszylinder
- 30: Pfeil
- 31: Ansatz
- 32: Nase
- 33: U-förmige Aufnahme
- 34: U-förmige Klammer
- 35: Schwenkachse
- 36: Stirnfläche
- 37: Aufnahmenut
- 38: Aufnahmenut
- 39: Zentriernut
- 40: Abflachung
- 41: Rastöffnung
- 42: Stellschraube
- 43: Rastkugel
- 44: Druckstange
- 45: proximales Ende der Druckstange
- 46: Druckelement
- 47: Gewindeabschnitt
- 48: Gewindebuchse
- 49: Befestigungselement
- 50: Führungsnut
- 51: Schraube
- 52: distales Ende der Druckstange
- 53: Kugel
- 54: Hohlraum
- 55: Auflaufschräge
- 56: Öffnung
- 57: umlaufender Ringwulst
- 58: Dichtung
- 59: Austrittsöffnung
- 60: Luer-Lock-Verbindung
- 61: Öffnung
- 62: Rückspülkanäle

## Patentansprüche

1. Vorrichtung zum Applizieren von Knochenzement mit einem Gehäuse (2), einer sich an das Gehäuse anschließenden Kammer (29) zur Aufnahme des Knochenzements, und mit einem in dem Gehäuse (2) längsverschiebbar angeordneten Schaft (8), an dessen distalen Ende ein innerhalb der Kammer (29) angeordneter Kolben (20) vorgesehen ist, durch den der Knochenzement durch eine in der Kammer (29) ausgebildete Austrittsöffnung (59) unter hohem Druck herauspressbar ist, wobei die Kammer (29) von dem Gehäuse (2) abnehmbar ausgebildet ist, an dem Gehäuse (2) eine Befestigungseinrichtung (7) zum lösbaren Befestigen der Kammer (29) vorgesehen ist und der Kolben (20) als von dem Schaft (8) getrenntes Teil ausgebildet ist,
**dadurch gekennzeichnet,**
**dass** an dem distalen Ende des Schafts (8) zur lösbaren Kopplung von Kolben (20) und Schaft (8) eine von einer Freigabestellung in eine Kopplungsstellung und zurück umstellbare Kopplungseinrichtung (16) vorgesehen ist, wobei in der Kopplungsstellung Kolben (20) und Schaft (8) zur gemeinsamen Längsverschiebung miteinander verkoppelt sind, während in der Freigabestellung Kolben (20) und Schaft (8) voneinander entkoppelbar sind.

2. Vorrichtung nach Anspruch 1,
**dadurch gekennzeichnet,**
**dass** in der Kopplungsstellung der Schaft (8) gegenüber dem Kolben (20) verdrehbar ist.

3. Vorrichtung nach Anspruch 1 oder 2,
**dadurch gekennzeichnet,**
**dass** in der Kopplungsstellung Kolben (20) und Schaft (8) im wesentlichen spielfrei in Verschieberichtung miteinander verkoppelt sind.

4. Vorrichtung nach irgendeinem der vorhergehenden Ansprüche,
**dadurch gekennzeichnet,**
**dass** der Kolben (20) ein insbesondere elastisches Dichtungselement (58) zur Abdichtung der Kolbenaußenseite gegenüber der Innenwand der Kammer (29) umfasst.

5. Vorrichtung nach Anspruch 4,
**dadurch gekennzeichnet,**
**dass** der Kolben (20) und das Dichtungselement (58) als getrennte Teile oder integral miteinander ausgebildet sind.

6. Vorrichtung nach irgendeinem der vorhergehenden Ansprüche,
**dadurch gekennzeichnet,**
**dass** der Kolben (20) zumindest in seinem mit der Kopplungseinrichtung (16) zusammenwirkenden Abschnitt starr ausgebildet ist.

7. Vorrichtung nach irgendeinem der vorhergehenden Ansprüche,
**dadurch gekennzeichnet,**
**dass** die Kopplung zwischen dem Kolben (20) und dem Schaft (8) durch eine oder mehrere in Verschieberichtung wirkende Hinterschneidungen (18, 19, 57) gebildet ist.

8. Vorrichtung nach irgendeinem der vorhergehenden Ansprüche,
**dadurch gekennzeichnet,**
**dass** zur Bildung der Kopplungseinrichtung (16) an dem distalen Ende des Schafts (8) Spreizklauen (17) ausgebildet sind, dass an dem proximalen Ende des Kolbens (20) zumindest eine Kopplungsöffnung (56) vorgesehen ist und dass die Spreizklauen (17) in die Kopplungsöffnung (56) einführbar sind und zur Kopplung mit dem Kolben (20) auseinanderspreizbar oder zusammenziehbar sind.

9. Vorrichtung nach Anspruch 8,
**dadurch gekennzeichnet,**
**dass** die auseinander gespreizte Stellung der Spreizklauen (17) der Kopplungsstellung der Kopplungseinrichtung (16) und die zusammengezogene Stellung der Freigabestellung entspricht.

10. Vorrichtung nach irgendeinem der vorhergehenden Ansprüche,
**dadurch gekennzeichnet,**
**dass** am proximalen Ende des Schafts (8) ein Betätigungsorgan (12) vorgesehen ist, über das die Kopplungseinrichtung (16) zwischen der Freigabestellung und der Kopplungsstellung verstellbar ist.

11. Vorrichtung nach Anspruch 10,
**dadurch gekennzeichnet,**
**dass** der Schaft (8) eine Längsbohrung (14) aufweist, durch die ein über das Betätigungsorgan (12) steuerbares Kraftübertragungselement (13) geführt ist, über das eine Betätigung des Betätigungsorgans (12), insbesondere eine Verdrehung des Betätigungsorgans (12), in eine Verstellung der Kopplungseinheit (16) umgesetzt wird.

12. Vorrichtung nach irgendeinem der vorhergehenden Ansprüche,
**dadurch gekennzeichnet,**
**dass** am proximalen Ende des Schafts (8) eine Handhabe (10) zur Längsverschiebung des Schafts (8) vorgesehen ist.

13. Vorrichtung nach irgendeinem der vorhergehenden Ansprüche,
**dadurch gekennzeichnet,**
**dass** die Kammer (29) zylinderförmig, insbesondere kreiszylinderförmig ausgebildet ist.

14. Vorrichtung nach irgendeinem der vorhergehenden Ansprüche,
**dadurch gekennzeichnet,**
**dass** die Kammer (29) drehfest an dem Gehäuse (2) befestigbar ist.

15. Vorrichtung nach irgendeinem der vorhergehenden Ansprüche,
**dadurch gekennzeichnet,**
**dass** die Befestigungseinrichtung (7) eine Aufnahme (37, 38) aufweist, in die das insbesondere komplementär zur der Aufnahme (37, 38) ausgebildete proximale Ende der Kammer (29) einsetzbar ist, so dass bei eingesetzter Kammer (29) sowohl eine in Längsrichtung der Verschiebung als auch eine konzentrisch zur Verschieberichtung wirkende kraftschlüssige Verbindung zwischen der Kammer (29) und dem Gehäuse (2) besteht.

16. Vorrichtung nach Anspruch 15,
**dadurch gekennzeichnet,**
**dass** das proximale Ende der Kammer (29) einen Ansatz (31) aufweist, der in die Aufnahme (37, 38) einsetzbar ist.

17. Vorrichtung nach irgendeinem der vorhergehenden Ansprüche,
**dadurch gekennzeichnet,**
**dass** der Schaft (8) durch eine Schraubbewegung innerhalb des Gehäuses (2) längsverschiebbar ist.

18. Vorrichtung nach Anspruch 17,
**dadurch gekennzeichnet,**
**dass** die Vorrichtung (1) zwischen der Verschiebung des Schafts (5) durch die Schraubbewegung und einer direkten Verschiebung in Längsrichtung ohne Schraubbewegung umstellbar ist.

19. Vorrichtung nach irgendeinem der vorhergehenden Ansprüche,
**dadurch gekennzeichnet,**
**dass** der Schaft (8) einen Eingriffsabschnitt mit einem Schraubgewinde (9) umfaßt, das in eine am Gehäuse (2) vorgesehene Gegenverzahnung (26) eingreift, so dass beim Verdrehen des Schafts (8) die Längsverschiebung des Kolbens (20) erfolgt.

20. Vorrichtung nach Anspruch 19,
**dadurch gekennzeichnet,**
**dass** das Schraubgewinde (9) und die Gegenverzahnung (26) voneinander lösbar sind.

21. Vorrichtung nach Anspruch 20,
**dadurch gekennzeichnet,**
**dass** zum Lösen die Gegenverzahnung (26) in einer Richtung im wesentlichen senkrecht zur Verschieberichtung des Kolbens (20) bewegbar ist.

22. Vorrichtung nach Anspruch 20 oder 21,
**dadurch gekennzeichnet,**
**dass** im gelösten Zustand der Schaft (8) im wesentlichen frei in dem Gehäuse (2) längsverschiebbar ist.

23. Vorrichtung nach irgendeinem der Ansprüche 19 bis 22,
**dadurch gekennzeichnet,**
**dass** die Gegenverzahnung (26) unter Vorspannung gegen das Schraubgewinde (9) gedrückt wird.

24. Vorrichtung nach irgendeinem der Ansprüche 19 bis 23,
**dadurch gekennzeichnet,**
**dass** in dem Gehäuse (2) ein Sperrelement (22) verschiebbar gelagert ist, dass das Sperrelement (22) eine Durchgangsöffnung (23) besitzt, durch die der Schaft (8) geführt ist, dass innerhalb der Durchgangsöffnung (23) die Gegenverzahnung (26) ausgebildet ist und dass bei einem Verschieben des Sperrelements (22) in einer Richtung das Schraubgewinde (9) mit der Gegenverzahnung (26) in Eingriff kommt, während bei einer Verschiebung des Sperrelements (22) in der entgegengesetzten Richtung das Schraubgewinde (9) und die Gegenverzahnung (26) außer Eingriff kommen und der Schaft (8) innerhalb der Durchgangsöffnung (23) frei längsverschiebbar ist.

25. Vorrichtung nach Anspruch 24,
**dadurch gekennzeichnet,**
**dass** das Sperrelement (22) zum Verschieben von außerhalb des Gehäuses (2) zugreifbar ist.

26. Vorrichtung nach Anspruch 25,
**dadurch gekennzeichnet,**
**dass** der der Gegenverzahnung (26) gegenüberliegende Bereich des Sperrelements (22) einen von außerhalb des Gehäuses (2) zugreifbaren Betätigungsabschnitt (24) bildet und dass bei Drücken auf den Betätigungsabschnitt (24) das Schraubgewinde (9) und die Gegenverzahnung (26) außer Eingriff geraten.

27. Vorrichtung nach irgendeinem der Ansprüche 19 bis 26,
**dadurch gekennzeichnet,**
**dass** die beim Applizieren des Knochenzements unter Druck aneinander anliegenden Zahnflanken der Gegenverzahnung (26) und/oder der Flanken des Gewindeprofils des Schraubgewindes (9) einen Winkel von kleiner oder gleich ca. 90° mit der zur Verschieberichtung parallel verlaufenden Längsachse (27) des Schafts (9) einschließen.

28. Vorrichtung nach Anspruch 27,
**dadurch gekennzeichnet,**
**dass** die anderen Zahnflanken der Gegenverzahnung (26) und/oder die anderen Flanke des Gewindeprofils des Schraubgewindes (9) einen Winkel von mehr als ca. 90° mit der zur Verschieberichtung parallel verlaufenden Längsachse (27) des Schafts (8) einschließen.

29. Vorrichtung nach irgendeinem der vorhergehenden Ansprüche,
**dadurch gekennzeichnet,**
**dass** an der Austrittsöffnung (59) der Kammer (29) ein Anschluss für eine Kanüle, insbesondere eine Luer-Lock-Verbindung (60) vorgesehen ist.

30. Vorrichtung nach irgendeinem der vorhergehenden Ansprüche,
**dadurch gekennzeichnet,**
**dass** alle während der Applikation mit Knochenzement in Berührung kommenden Teile als Wegwerfartikel ausgebildet sind.

31. Vorrichtung nach irgendeinem der vorhergehenden Ansprüche,
**dadurch gekennzeichnet,**
**dass** alle während der Applikation nicht mit Knochenzement in Berührung kommenden Teile der Vorrichtung als mehrmals verwendbare Artikel ausgebildet sind.

32. Vorrichtung nach irgendeinem der vorhergehenden Ansprüche,
**dadurch gekennzeichnet,**
**dass** die Kammer (29) und/oder der Kolben (20) aus Kunststoff bestehen.

33. Vorrichtung nach irgendeinem der vorhergehenden Ansprüche,
**dadurch gekennzeichnet,**
**dass** das Gehäuse (2) und/oder der Schaft (8) aus Metall bestehen.

34. Vorrichtung nach irgendeinem der vorhergehenden Ansprüche, bei der die Kammer (29) von dem Gehäuse (2) abgenommen ist.

## Claims

1. An apparatus for the application of bone cement having a housing (2), a chamber (29) adjoining the housing for the reception of the bone cement and having a shaft (8) which is longitudinally displaceably arranged in the housing (2) and at whose distal end a piston (20) arranged inside the chamber (29) is provided by which the bone cement can be pressed out under high pressure through an outlet opening (59) formed in the chamber (29), wherein the chamber (29) is made to be removable from the housing (2), wherein a fastening device (7) is provided at the housing (2) for the releasable fastening of the chamber (29) and wherein the piston (20) is made as a part separate from the shaft (8),
**characterised in that**
a coupling device (16) which can be changed from a release position into a coupling position and back is provided at the distal end of the shaft (8) for the releasable coupling of the piston (20) and the shaft (8), with the piston (20) and the shaft (8) being coupled together in the coupling position for joint longitudinal displacement, whereas the piston (20) and the shaft (8) can be decoupled from one another in the release position.

2. An apparatus in accordance with claim 1, **characterised in that** the shaft (8) is rotatable with respect to the piston (20) in the coupling position.

3. An apparatus in accordance with claim 1 or claim 2, **characterised in that** the piston (20) and the shaft (8) are coupled to one another substantially free of play in the displacement direction in the coupling position.

4. An apparatus in accordance with any one of the preceding claims, **characterised in that** the piston (20) includes a sealing element (58), in particular a resilient sealing element, for the sealing of the outer side of the piston with respect to the inner wall of the chamber (29).

5. An apparatus in accordance with claim 4, **characterised in that** the piston (20) and the sealing element (58) are made as separate parts or integrally with one another.

6. An apparatus in accordance with any one of the preceding claims, **characterised in that** the piston (20) is made rigid at least in its section cooperating with the coupling device (16).

7. An apparatus in accordance with any one of the preceding claims, **characterised in that** the coupling between the piston (20) and the shaft (8) is formed by one or more undercuts (18, 19, 57) operative in the displacement direction.

8. An apparatus in accordance with any one of the preceding claims, **characterised in that** spreading claws (17) are formed at the distal ends of the shaft (8) for the formation of the coupling device (16); **in that** at least one coupling opening (56) is provided at the proximal end of the piston (20); and **in that** the spreading claws (17) can be introduced into the coupling opening (56) and can be spread apart or drawn together for the coupling with the piston (20).

9. An apparatus in accordance with claim 8, **characterised in that** the spread apart position of the spreading claws (17) corresponds to the coupling position of the coupling device (16) and the drawn together position corresponds to the release position.

10. An apparatus in accordance with any one of the preceding claims, **characterised in that** an actuation member (12) is provided at the proximal end of the shaft (8) via which the coupling device (16) can be adjusted between the release position and the coupling position.

11. An apparatus in accordance with claim 10, **characterised in that** the shaft (8) has a longitudinal bore (14) through which a force transmission element (13) can be guided which is controllable via an actuation member (12) and via which an actuation of the actuation member (12), in particular a rotation of the actuation member (12), is converted into an adjustment of the coupling unit (16).

12. An apparatus in accordance with any one of the preceding claims, **characterised in that** a handle (10) is provided at the proximal end of the shaft (8) for the longitudinal displacement of the shaft (8).

13. An apparatus in accordance with any one of the preceding claims, **characterised in that** the chamber (29) is cylindrical, in particular circularly cylindrical.

14. An apparatus in accordance with any one of the preceding claims, **characterised in that** the chamber (29) can be fastened rotationally fixedly to the housing (2).

15. An apparatus in accordance with any one of the preceding claims, **characterised in that** the fastening device (7) has a receiver (37, 38) into which the proximal end of the chamber (29) can be inserted, said proximal end in particular being made complementary to the receiver (37, 38), such that, when the chamber (29) is inserted, a force transmitting connection is present between the chamber (29) and the housing (2) which is operative both in the longitudinal direction of the displacement and concentrically to the direction of displacement.

16. An apparatus in accordance with claim 15, **characterised in that** the proximal end of the chamber (29) has a nose (31) which can be inserted into the receiver (37, 38).

17. An apparatus in accordance with any one of the preceding claims, **characterised in that** the shaft (8) is longitudinally displaceable by a screw movement inside the housing (2).

18. An apparatus in accordance with claim 17, **characterised in that** the apparatus (1) can be changed over between the displacement of the shaft (5) by the screw movement and a direct displacement in the longitudinal direction without a screw movement.

19. An apparatus in accordance with any one of the preceding claims, **characterised in that** the shaft (8) includes an engagement section with a screw thread (9) which engages into a counter tooth arrangement (26) provided at the housing (2) such that the longitudinal displacement of the piston (20) takes place on the rotation of the shaft (8).

20. An apparatus in accordance with claim 19, **characterised in that** the screw thread (9) and the counter tooth arrangement (26) can be released from one another.

21. An apparatus in accordance with claim 20, **characterised in that** the counter tooth arrangement (26) is movable in a direction substantially perpendicular to the direction of displacement of the piston (20) for the release.

22. An apparatus in accordance with claim 20 or claim 21, **characterised in that** the shaft (8) is substantially freely displaceable in the housing (2) in the released state.

23. An apparatus in accordance with any one of the claims 19 to 22, **characterised in that** the counter tooth arrangement (26) is pressed towards the screw thread (9) under pre-tension.

24. An apparatus in accordance with any one of the claims 19 to 23, **characterised in that** a blocking element (22) is displaceably supported in the housing (2); **in that** the blocking element (22) has a passage opening (23) through which the shaft (8) is guided; **in that** the counter tooth arrangement (26) is formed inside the passage opening (23); and **in that**, on a displacement of the blocking element (22) in one direction, the screw thread (9) comes into engagement with the counter tooth arrangement (26), whereas, on a displacement of the blocking element (22) in the opposite direction, the screw thread (9) and the counter tooth arrangement (26) come out of engagement and the shaft (8) is freely longitudinally displaceable inside the passage opening (23).

25. An apparatus in accordance with claim 24, **characterised in that** the blocking element (22) is accessible from outside the housing (2) for the displacement.

26. An apparatus in accordance with claim 25, **characterised in that** the region of the blocking element (22) disposed opposite the counter ' tooth arrangement (26) forms an actuation section (24) which can be accessed from outside the housing (2); and **in that**, on a pressing onto the actuation section (24), the screw thread (9) and the counter tooth arrangement (26) come out of engagement.

27. An apparatus in accordance with any one of the claims 19 to 26, **characterised in that** the tooth flanks of the counter tooth arrangement (26) contacting one another on the application of the bone cement under pressure and/or the flanks of the thread profile of the screw thread (9) include an angle of less than or equal to approximately 90° with the longitudinal axis (27) of the shaft (9) extending parallel to the direction of displacement.

28. An apparatus in accordance with claim 27, **characterised in that** the other tooth flanks of the counter tooth arrangement (26) and/or the other flanks of the thread profile of the screw thread (9) include an angle of more than approximately 90° with the longitudinal axis (27) of the shaft (8) extending parallel to the direction of displacement.

29. An apparatus in accordance with any one of the preceding claims, **characterised in that** a connection for a cannula, in particular a luer lock connection (60), is provided at the outlet opening (59) of the chamber (29).

30. An apparatus in accordance with any one of the preceding claims, **characterised in that** all parts coming into contact with bone cement during the application are made as disposable articles.

31. An apparatus in accordance with any one of the preceding claims, **characterised in that** all parts of the apparatus not coming into contact with bone cement during the application are made as articles which can be used several times.

32. An apparatus in accordance with any one of the preceding claims, **characterised in that** the chamber (29) and/or the piston (20) is/are made of plastic.

33. An apparatus in accordance with any one of the preceding claims, **characterised in that** the housing (2) and/or the shaft (8) is/are made of metal.

34. An apparatus in accordance with any one of the preceding claims, wherein the chamber (29) is removed from the housing (2).

## Revendications

1. Dispositif d'application de ciment osseux, comportant un boîtier (2), une chambre (29) se raccordant au boîtier pour recevoir le ciment osseux, et comportant un fût (8) agencé à déplacement longitudinal dans le boîtier (2), sur l'extrémité distale duquel est prévu un piston (20) agencé à l'intérieur de la chambre (29), par lequel le ciment osseux peut être pressé sous haute pression à travers une ouverture de sortie (59) réalisée dans la chambre (29), la chambre (29) étant réalisée amovible du boîtier (2), sur le boîtier (2) étant prévu un système de fixation (7) pour fixer de manière détachable la chambre (29) et le piston (20) étant réalisé sous forme de partie séparée du fût (8),
**caractérisé en ce que**
à l'extrémité distale du fût (8), il est prévu un système d'accouplement (16) commutable depuis une position de libération jusque dans une position d'accouplement et inversement, pour accoupler de manière détachable le piston (20) et le fût (8), et dans la position d'accouplement, le piston (20) et le fût (8) sont accouplés l'un à l'autre en vue d'un déplacement longitudinal commun, tandis que dans la position de libération, le piston (20) et le fût (8) peuvent être découplés l'un de l'autre.

2. Dispositif selon la revendication 1, **caractérisé en ce que** dans la position d'accouplement, le fût (8) peut être tourné par rapport au piston (20).

3. Dispositif selon la revendication 1 ou 2, **caractérisé en ce que** dans la position d'accouplement, le piston (20) et le fût (8) sont accouplés l'un à l'autre sensiblement sans jeu en direction de déplacement.

4. Dispositif selon l'une des revendications précédentes, **caractérisé en ce que** le piston (20) comprend un élément d'étanchéité (58) en particulier élastique pour étancher le côté extérieur du piston par rapport à la paroi intérieure de la chambre (29).

5. Dispositif selon la revendication 4, **caractérisé en ce que** le piston (20) et l'élément d'étanchéité (58) sont réalisés en tant que parties séparées ou de manière intégrale l'un avec l'autre.

6. Dispositif selon l'une des revendications précédentes, **caractérisé en ce que** le piston (20) est réalisé rigide au moins dans son tronçon coopérant avec le système d'accouplement (16).

7. Dispositif selon l'une des revendications précédentes, **caractérisé en ce que** l'accouplement entre le piston (20) et le fût (8) est formé par une ou plusieurs contre-dépouilles (18, 19, 57) agissant en direction de déplacement.

8. Dispositif selon l'une des revendications précédentes, **caractérisé en ce que** des mâchoires d'écartement (17) sont réalisées pour former le système d'accouplement (16) sur l'extrémité distale du fût (8), **en ce qu'**à l'extrémité proximale du piston (20) est prévue au moins une ouverture d'accouplement (56) et **en ce que** les mâchoires d'écartement (17) peuvent être introduites dans l'ouverture d'accouplement (56) et peuvent être écartées ou resserrées pour l'accouplement avec le piston (20).

9. Dispositif selon la revendication 8, **caractérisé en ce que** la position écartée des mâchoires d'écartement (17) correspond à la position d'accouplement du système d'accouplement (16) et la position resserrée correspond à la position de libération.

10. Dispositif selon l'une des revendications précédentes, **caractérisé en ce qu'**à l'extrémité proximale du fût (8) est prévu un organe d'actionnement (12) via lequel le dispositif d'accouplement (16) peut être réglé entre la position de libération et la position d'accouplement.

11. Dispositif selon la revendication 10, **caractérisé en ce que** le fût (8) présente un perçage longitudinal (14) à travers lequel est guidé un élément de transmission de forces (13) qui peut être commandé via l'organe d'actionnement (12) et via lequel un actionnement de l'organe d'actionnement (12), en particulier une rotation de l'organe d'actionnement (12), est converti en un réglage de l'unité d'accouplement (16).

12. Dispositif selon l'une des revendications précédentes, **caractérisé en ce qu'**à l'extrémité proximale du fût (8) est prévue une manette (10) pour le déplacement longitudinal du fût (8).

13. Dispositif selon l'une des revendications précédentes, **caractérisé en ce que** la chambre (29) est réalisée de forme cylindrique, en particulier de forme cylindrique circulaire.

14. Dispositif selon l'une des revendications précédentes, **caractérisé en ce que** la chambre (29) peut être fixée solidaire en rotation sur le boîtier (2).

15. Dispositif selon l'une des revendications précédentes, **caractérisé en ce que** le système de fixation (7) présente un logement (37, 38) dans lequel peut être insérée l'extrémité proximale de la chambre (29), qui est réalisée en particulier complémentaire au logement (37, 38), de sorte que lorsque la chambre (29) est insérée, on obtient une liaison par coopération de forces entre la chambre (29) et le boîtier (2), agissant tant en direction longitudinale de déplacement que concentriquement par rapport à la direction de déplacement.

16. Dispositif selon la revendication 15, **caractérisé en ce que** l'extrémité proximale de la chambre (29) présente un embout (31) qui peut être inséré dans le logement (37, 38).

17. Dispositif selon l'une des revendications précédentes, **caractérisé en ce que** le fût (8) est déplaçable en longueur par un mouvement de vissage à l'intérieur du boîtier (2).

18. Dispositif selon la revendication 17, **caractérisé en ce que** le dispositif (1) peut être commuté entre le déplacement du fût (5) par le mouvement de vissage et un déplacement direct en direction longitudinale sans mouvement de vissage.

19. Dispositif selon l'une des revendications précédentes, **caractérisé en ce que** le fût (8) comprend un tronçon d'engagement avec un filetage (9) qui s'engage dans une denture antagoniste (26) sur le boîtier (2), de telle sorte que le déplacement longitudinal du piston (20) a lieu lorsqu'on fait tourner le fût (8).

20. Dispositif selon la revendication 19, **caractérisé en ce que** le filetage (9) et la denture antagoniste (26) peuvent être détachés l'un de l'autre.

21. Dispositif selon la revendication 20, **caractérisé en ce que** pour effectuer le détachement, la denture antagoniste (26) peut être déplacée dans une direction sensiblement perpendiculaire à la direction de déplacement du piston (20).

22. Dispositif selon la revendication 20 ou 21, **caractérisé en ce qu'**à l'état de détachement, le fût (8) est sensiblement librement déplaçable en longueur dans le boîtier (2).

23. Dispositif selon l'une des revendications 19 à 22, **caractérisé en ce que** la denture antagoniste (26) est pressée sous précontrainte contre le filetage (9).

24. Dispositif selon l'une des revendications 19 à 23, **caractérisé en ce que** dans le boîtier (2), un élément de blocage (22) est monté à déplacement, **en ce que** l'élément de blocage (22) possède une ouverture de passage (23) à travers laquelle est guidé le fût (8), **en ce qu'**à l'intérieur de l'ouverture de passage (23) est réalisée la denture antagoniste (26) et **en ce que** lors d'un déplacement de l'élément de blocage (22) dans une direction, le filetage (9) vient en engagement avec la denture antagoniste (26) tandis que lors d'un déplacement de l'élément de blocage (22) dans la direction opposée, le filetage (9) et la denture antagoniste (26) viennent hors d'engagement, et le fût (8) peut être déplacé librement en longueur à l'intérieur de l'ouverture de passage (23).

25. Dispositif selon la revendication 24, **caractérisé en ce que** pour le déplacement, l'élément de blocage (22) est saisissable depuis l'extérieur du boîtier (2).

26. Dispositif selon la revendication 25, **caractérisé en ce que** la région opposée à la denture antagoniste (26), de l'élément de blocage (22), forme un tronçon d'actionnement (24) saisissable depuis l'extérieur du boîtier (2) et **en ce que** lorsqu'on appuie sur le tronçon d'actionnement (24), le filetage (9) et la denture antagoniste (26) parviennent hors d'engagement.

27. Dispositif selon l'une des revendications 19 à 26, **caractérisé en ce que** les flancs de dents de la denture antagoniste (26), qui sont en appui sous pression les uns sur les autres lors de l'application du ciment osseux, et/ou les flancs du profil de filetage du filetage (9) forment un angle inférieur ou égal à environ 90° avec l'axe longitudinal (27) du fût (8), qui s'étend parallèlement à la direction de déplacement.

28. Dispositif selon la revendication 27, **caractérisé en ce que** les autres flancs de dents de la denture antagoniste (26) et/ou les autres flancs du profil de filetage du filetage (9) forment un angle supérieure à environ 90° avec l'axe longitudinal (27) du fût (9), qui s'étend parallèlement à la direction de déplacement.

29. dispositif selon l'une des revendications précédentes, **caractérisé en ce qu'**au niveau de l'ouverture de sortie (59) de la chambre (29) est prévu un raccord pour une canule, en particulier pour une jonction de Luer-Lock (60).

30. Dispositif selon l'une des revendications précédentes, **caractérisé en ce que** toutes les parties qui viennent en contact avec le ciment osseux pendant l'application sont réalisées en tant qu'articles jetables.

31. Dispositif selon l'une des revendications précédentes, **caractérisé en ce que** toutes les parties qui ne viennent pas en contact avec le ciment osseux pendant l'application sont réalisées en tant qu'articles réutilisables plusieurs fois.

32. Dispositif selon l'une des revendications précédentes, **caractérisé en ce que** la chambre (29) et/ou le piston (20) sont en matière plastique.

33. Dispositif selon l'une des revendications précédentes, **caractérisé en ce que** le boîtier (2) et/ou le fût (8) sont en métal.

34. Dispositif selon l'une des revendications précédentes, dans lequel la chambre (29) est enlevée du boîtier (2).
